(19) Europäisches Patentamt
European Patent Office
Office européen des brevets

(11) **EP 2 133 075 A1**

(12) **EUROPEAN PATENT APPLICATION**

(43) Date of publication:
**16.12.2009 Bulletin 2009/51**

(51) Int Cl.:
*A61K 31/343* (2006.01)   *A61P 9/06* (2006.01)

(21) Application number: **08290532.4**

(22) Date of filing: **10.06.2008**

(84) Designated Contracting States:
**AT BE BG CH CY CZ DE DK EE ES FI FR GB GR
HR HU IE IS IT LI LT LU LV MC MT NL NO PL PT
RO SE SI SK TR**
Designated Extension States:
**AL BA MK RS**

(71) Applicant: **Sanofi-Aventis**
**75013 Paris (FR)**

(72) Inventors:
• **Gaudin, Christophe**
**75013 Paris (FR)**

• **Hamdani, Nacéra**
**75013 Paris (FR)**
• **Radzik, David**
**75013 Paris (FR)**
• **Van Eickels, Martin**
**65926 Frankfurt am Main (DE)**

(74) Representative: **Romanowski, Caroline et al**
**Sanofi-Aventis**
**174 avenue de France**
**75013 Paris (FR)**

(54) **Use of dronedarone for the preparation of a medicament intended for the prevention of cardioversion**

(57)    Use of dronedarone for the preparation of a medicament intended for the prevention of cardioversion.

EP 2 133 075 A1

**Description**

The instant invention relates to the use of dronedarone for the preparation of a medicament intended for the prevention of cardioversion especially electrical cardioversion.

2-n-butyl-3-[4-(3-di-n-butylaminopropoxy)benzoyl]-5-methylsulfonamido-benzofuranne or dronedarone and its pharmaceutically acceptable salts are described in European patent EP 0 471 609 B1.

Dronedarone is a multi-channel blocker that affects calcium, potassium and sodium channels and has anti-adrenergic properties.

Dronedarone is an anti-arrhythmic agent for the treatment of patients with a history of atrial fibrillation or atrial flutter.

Electrical therapy has been used to treat a variety of conditions since the 18th century and has led to the development of trans-thoracic direct current electrical cardioversion. It is now the treatment of choice for many cardiac arrhythmias.

Trans-thoracic electrical cardioversion has become the standard method for terminating AF since Lown first described it in 1962 (Lown B, Amarasingham R, Neuman J, et al: New method for treating cardiac arrhythmias; Use of synchronized capacitor discharge. JAMA 182: 548- 555, 1962). Since then, this technique has been widely used and shown too effective. Research over the last decade has resulted in a better understanding of the mechanisms of defibrillation, the development of new technologies and energy waveforms, and novel optimization strategies to improve efficacy rates, patient safety, and success in refractory cases.

Electrical transthoracic cardioversion terminates arrhythmia by the delivery of a synchronized shock applied on the patient's chest via two paddles, it depolarizes the tissue involved in a reentrant circuit responsible for the arrhythmia. Depolarization of all involved excitable tissue of the circuit makes the tissue refractory, which is no longer able to propagate or sustain reentry.

According to the "Critical Mass Hypothesis" high defibrillation energy levels can eliminate fibrillatory activity. This theory hypothesizes that; atrial or ventricular fibrillation is sustained by a certain amount of myocardium and terminated when the entire myocardium is uniformly depolarized (Zipes DP, Fischer J, King RM: Termination of ventricular fibrillation in dogs by depolarizing a critical amount of myocardium. Am J Cardiol 36:37- 44, 1975).

The success of electrical cardioversion for the treatment of atrial fibrillation can be as high as 87%, it depends on the patient's conditions in particular the duration of the atrial fibrillation episode as well as on intervention modalities. The position of the paddles used to apply the shock is particularly important, the anterior-lateral position with one paddle over the cardiac apex and one right infra-clavicular is the most effective (Botto GL, Politi A, Bonini W, et al: External cardioversion of atrial fibrillation: Role of paddle position on technical efficacy and energy requirements. Heart 82: 726-730, 1999).

Although effective the electrical cardioversion procedure can lead to sometimes severe complications in particular thromboembolic events such as stroke and lifethreatening cardiac arrhythmias.

Thromboembolic complications were reported in 1-7% of patients in particular in the absence of adequate anticoagulation (Bjerkelund CJ, Orning OM: The efficacy of anticoagulant therapy in preventing embolism related to DC electrical conversion of atrial fibrillation. Am J Cardiol 23:208-216, 1969), (Arnold AZ, Mick MJ, Mazurek RP, et al: Role of prophylactic anticoagulation for direct current cardioversion in patients with atrial fibrillation or atrial flutter. J Am Coll Cardiol 19:851-855, 1992).

Various arrhythmias may arise after cardioversion of atrial fibrillation, especially ventricular and supraventricular premature beats, bradycardia, and short periods of sinus arrest (Rabbino MD, et al: Complications and limitations of direct current countershock. JAMA 190:417- 420, 1964). Electrolyte imbalances or treatment with digitalis may precipitate other dangerous arrhythmias, such as ventricular tachycardia and fibrillation (Lown B, Likoff W, Dreifus LS: Cardioversion and digitalis drugs: Changed threshold to electric shock in digitalized animals. Circ Res 17:519- 531, 1965), (Aberg H, Cullhed I: Direct current countershock complications. Acta Med Scand 183:415- 421, 1968).

Skin burns are also a frequent complication of electrical cardioversion (Pagan-Carlo, Stone MS, Kerber RE: Nature and determinants of skin burns after transthoracic cardioversion. Am J Cardiol 79:689- 691, 1997).

Myocardial injury can also occur during electrical cardioversion (Lipkin DP, Frenneaux M, Stewart R, et al: Delayed improvement in exercise capacity after cardioversion of atrial fibrillation to sinus rhythm. Br Heart J 59:572-577, 1988), (Patton JN, Allen JD, Pantridge JF: The effects of shock energy, propranolol, and verapamil on cardiac damage caused by transthoracic countercheck. Circulation 69:357-368, 1984).

Furthermore electrical cardioversion is done under general anesthesia which by itself can lead to complications.

In conclusion electrical cardioversion is commonly used for the treatment of atrial fibrillation. Although a highly successful therapy, it often needs to be repeated because of atrial fibrillation recurrences which can occur at any time. Each electrical cardioversion procedure carries a risk of complications which can be severe such as stroke or lifethreatening cardiac arrhythmias.

For these reasons an agent which would decrease the need for electrical cardioversion would be of great benefit to atrial fibrillation patients.

The Inventors have now found that dronedarone decrease the need for cardioversion especially electrical cardioversion.

The subject of the instant invention is the use of dronedarone or one of its pharmaceutically acceptable salts for the preparation of a medicament intended to the prevention of cardioversion in patients with a history of atrial fibrillation or atrial flutter.

More precisely, the invention relates to the use of dronedarone or one of its pharmaceutically acceptable salts for the preparation of a medicament intended to the prevention of electrical cardioversion in patients with a history of atrial fibrillation or atrial flutter.

More precisely, the invention relates to the use of dronedarone or one of its pharmaceutically acceptable salts for the preparation of a medicament intended to the prevention of about 32% of cardioversion in patients with a history of atrial fibrillation or atrial flutter.

The percentage above corresponds to an average.

"Prevention of about 32%" means that a patient treated with dronedarone has a 32% lower risk of having a cardioversion compared to a patient not treated with dronedarone.

Among the pharmaceutically acceptable salts of dronedarone, mention may be made of the hydrochloride.

The expression « with a history of atrial fibrillation or atrial flutter » means a patient who has a history of atrial fibrillation (AF) or atrial flutter (AFL) and who can be either in sinus rhythm or in atrial fibrillation or atrial flutter at the time of dronedarone administration.

Among patients with a history of atrial fibrillation or atrial flutter, mention may be made of patients who further have at least one of the following risk factors :

- hypertension,
- diabete,
- prior cerebrovascular accident or systemic embolism,
- left atrium diameter greater that or equal to 50 mm by echocardiography,
- left ventricular ejection fraction less than 40% by 2D-echocardiography.

Among patients with a history of atrial fibrillation or atrial flutter, mention may also be made of patients having additional risk factors corresponding to at least one of the following diseases:

- hypertension,
- structural heart disease,
- tachycardia,
- coronary heart disease,
- non-rheumatic valvular heart disease,
- ischemic dilated cardiomyopathy,
- a history of ablation for AF/AFL for example catheter ablation or surgical ablation,
- supra-ventricular tachycardia other than AF/AFL,
- history of cardiac valve surgery,
- non-ischemic dilated cardiomyopathy,
- hypertrophic cardiomyopathy,
- rheumatic valvular heart disease,
- sustained ventricular tachycardia,
- congenital heart disease,
- a history of ablation for other reason than AF/AFL for example catheter ablation,
- ventricular fibrillation,

and/or at least a cardiac device chosen among:

- a pacemaker,
- an implanted cardioverter defibrillator.

Another object of the invention is a pharmaceutical composition which comprises, as active principle, a compound of formula (I) according to the present invention. This pharmaceutical composition comprises an effective dose of at least one compound of formula (I) according to the invention, or an addition salt thereof with a pharmaceutically acceptable salt, or a hydrate or solvate thereof, and at least one pharmaceutically acceptable excipient. Said excipients are chosen according to the pharmaceutical form and the administration route desired, among usual excipients known to one of skill in the art.

In the pharmaceutical compositions according to the invention for the oral, sublingual, sub-cutaneous, intramuscular, intra-venous, topical, local, intratracheal, intranasal, transdermal or rectal administration, the active principle of formula (I) above or its salt, solvate or hydrate, can be administered as a unitary dosage form, in blend with usual pharmaceutical excipients, to animals and human beings for the prevention or for the treatment of pathological states mentioned above. The appropriate unitary dosage forms comprise the oral forms, such as tablets, hard or soft gelatin capsules, powders, granules and oral solutions or suspensions, the sublingual, buccal, intratracheal, intraocular, intranasal forms, the forms adapted to inhalation, topical, transdermal, sub-cutaneous, intramuscular or intra-venous delivery, the rectal forms and the implants. For the topical application, the compounds of the invention may be used as creams, gels, ointments or lotions.

For its use in therapeutics, dronedarone and its pharmaceutically acceptable salts are incorporated in pharmaceuticals compositions.

These pharmaceutical compositions comprise an effective dose of at least dronedarone or one of its pharmaceutically acceptable salts and at least one pharmaceutically acceptable excipient.

Said excipients are chosen according to the pharmaceutical form and the administration route desired, among usual excipients known of one of skill in the art.

In the pharmaceutical compositions for the oral, sublingual, sub-cutaneous, intramuscular, intra-venous, topical, local, intratracheal, intranasal, transdermal or rectal administration, dronedarone or one of its pharmaceutically acceptable salts, can be administered as a unitary dosage form, in blend with usual pharmaceutical excipients, to animals and human in diseases above mentioned.

The appropriate unitary dosage forms comprise the oral forms, such as tablets, hard or soft gelatin capsules, powders, granules and oral solutions or suspensions, the sublingual, buccal, intratracheal, intraocular, intranasal forms, by inhalation, the topical, transdermal, sub-cutaneous, intramuscular or intra-venous forms, the rectal forms and the implants. For the topical application, the compounds of the invention may be used as creams, gels, ointments or lotions.

As an example, a unitary dosage form for dronedarone or one of its pharmaceutically acceptable salts, in the form of a tablet, can comprise the following ingredients:

| Ingredients | mg |
|---|---|
| dronedarone hydrochloride (corresponding to 400 mg of base) | 426 |
| Methylhydroxypropylcellulose | 21,1 |
| Lactose monohydrate | 46,55 |
| Modified corn starch | 45,5 |
| Polyvinylpyrrolidone | 65 |
| Poloxamer 407 | 40 |
| Anhydrous colloidal silica | 2,6 |
| magnesium stearate | 3,25 |
| | 650 |

| Ingredients | mg |
|---|---|
| dronedarone hydrochloride (corresponding to 400 mg of base) | 426 |
| microcrystalline cellulose | 65 |
| Anhydrous colloidal silica | 2,6 |
| anhydrous lactose | 42,65 |
| Polyvinylpyrrolidone | 13 |
| Poloxamer 407 | 40 |
| Macrogol 6000 | 57,5 |
| magnesium stearate | 3,25 |

(continued)

| Ingredients | mg |
| --- | --- |
| | 650 |

| Ingredients | mg |
| --- | --- |
| dronedarone hydrochloride (corresponding to 400 mg of base) | 426 |
| microcrystalline cellulose | 26 |
| corn starch | 45,5 |
| Polyvinylpyrrolidone | 65 |
| Poloxamer 407 | 40 |
| Anhydrous colloidal silica | 3,25 |
| magnesium stearate | 3,25 |
| Lactose monohydrate | 41,65 |
| | 650 |

| Ingredients | mg |
| --- | --- |
| dronedarone hydrochloride (corresponding to 400 mg of base) | 213 |
| microcrystalline cellulose | 13 |
| corn starch | 22,75 |
| Polyvinylpyrrolidone | 32,5 |
| Poloxamer 407 | 20 |
| Anhydrous colloidal silica | 1,3 |
| magnesium stearate | 1,625 |
| Lactose monohydrate | 20,825 |
| | 650 |

For oral administration, dronedarone daily dose may reach 800 mg.

In specific cases, higher or lower dosages may be appropriated; these dosages are comprised within the scope of the present invention. According to usual practice, the dosage suitable to each patient is determined by the physician according to the administration route, the weight, the disease, the body surface, the cardiac output and response of the patient.

The instant invention also relates to a method of treatment of the above mentioned disease which comprises the administration to a patient of an effective dose of at least dronedarone or one of its pharmaceutically acceptable salts.

The invention is illustrated with the above data with reference to the following figure:

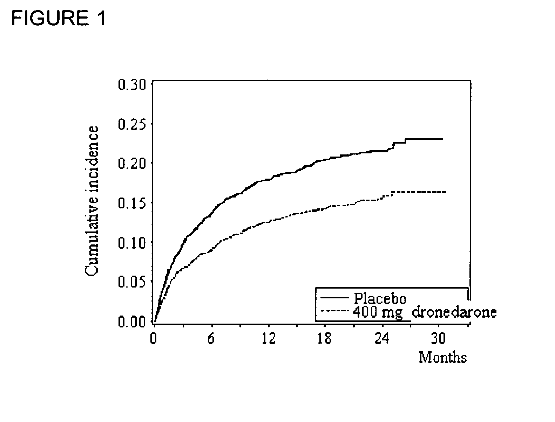

Figure 1 represents Kaplan Meier cumulative incidence curves from randomization to first cardioversion on-study period of 30 months.

Efficacy of dronedarone and its pharmaceutically acceptable salts versus placebo for the prevention of cardioversion was provided via dronedarone hydrochloride during a prospective, multinational, double-blind, randomized, multi-center, placebo-controlled, parallel group trial.

**I. Selection of patients**

Patients must have a history of atrial fibrillation or atrial flutter and/or may be in normal sinus rhythm or in atrial fibrillation or flutter at the time of recruitment.

Recruitment of patients was conducted taking into account the following inclusion criteria:

Inclusion criteria:

1) One of the following risk factors must be present:

- Age equal or greater that 70 years, or above 75 years, associated or not with at least one of the following risk factors :

   o hypertension (taking antihypertensive drugs of at least two different classes),
   o diabetes,
   o prior cerebrovascular accident (stroke or transient ischemic attack) or systemic embolism,
   o left atrium diameter greater that or equal to 50 mm by echocardiography,
   o left ventricular ejection fraction less than 40% by 2D-echocardiography,

2) availability of one electrocardiogram within the last six months, showing that the patients was or is in atrial fibrillation/flutter,

3) availability of one electrocardiogram within the last six months, showing that the patients was or is in sinus rhythm.

**II. Duration and treatment**

Study drug treatment units (placebo or dronedarone hydrochloride corresponding to 400 mg of base) were such that each patient took one tablet in the morning during or shortly after breakfast and one tablet in the evening during or shortly after dinner.

The treatment duration depended on the time of recruitment of each patient in the trial and could be comprised from 12 months to 30 months.

**III. Results**

Results were calculated using non-parametric Kaplan-Meier estimate.

Cox's proportional hazard model was used to estimate the hazard ratio also called relative risk.

Relative risk (RR) is the ratio between the risk of having a cardioversion for patients treated with dronedarone and the risk of having a cardioversion for patients treated with placebo.

Percentage of decrease of an event is calculated as follow:

$$x = 1 - RR.$$

Results relating to cardioversion

From the 4628 patients included in the trial, 2301 were part of the group treated with dronedarone hydrochloride.

481 events were registered in the placebo group versus 339 in the group treated with dronedarone hydrochloride.

Calculated relative risk was equal to 0.68, i.e. a decrease of electrical cardioversion of 32% (p <0.001).

Figure 1 shows that the effect of dronedarone occurred early and increased over time.

**Claims**

**1.** Use of dronedarone for the preparation of a medicament intended for the prevention of cardioversion.

**2.** Use according to claim 1 for the preparation of a medicament intended for the prevention of electrical cardioversion.

**3.** Use according to claim 1 or for the preparation of a medicament intended for the prevention of about 32% of cardioversion.

**4.** Use according to claim 1, 2 or 3, **characterized in that** the patients have a history of atrial fibrillation or atrial flutter.

**5.** Use according to one of the previous claims, **characterized in that** the patients have at least one of the following risk factors:

- hypertension,
- diabete,
- prior cerebrovascular accident or systemic embolism,
- left atrium diameter greater that or equal to 50 mm by echocardiography,
- left ventricular ejection fraction less than 40% by 2D-echocardiography.

**6.** Use according to one of the previous claims, **characterized in that** the patients have additional risk factors corresponding to at least one of the following diseases:

- hypertension,
- structural heart disease,
- tachycardia,
- coronary heart disease,
- non-rheumatic valvular heart disease,
- ischemic dilated cardiomyopathy,
- ablation for AF/AFL,
- supra-ventricular tachycardia other than AF/AFL,
- history of cardiac valve surgery,
- non-ischemic dilated cardiomyopathy,
- hypertrophic cardiomyopathy,
- rheumatic valvular heart disease,
- sustained ventricular tachycardia,
- congenital heart disease,
- ablation for other reason than AF/AFL,
- ventricular fibrillation,

and/or at least a cardiac device chosen among:

- a pacemaker,
- an implanted cardioverter defibrillator.

**7.** Use according to one of the preceding claims, **characterized in that,** for oral administration, dronedarone daily dose may reach 800 mg.

FIGURE 1

Europäisches
Patentamt

European
Patent Office

Office européen
des brevets

# EUROPEAN SEARCH REPORT

Application Number

EP 08 29 0532

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | HOHNLOSER STEFAN H ET AL: "Rationale and design of ATHENA: A placebo-controlled, double-blind, parallel arm Trial to assess the efficacy of dronedarone 400 mg bid for the prevention of cardiovascular Hospitalization or death from any cause in patiENts with Atrial fibrillation/atrial flutter." JOURNAL OF CARDIOVASCULAR ELECTROPHYSIOLOGY JAN 2008, vol. 19, no. 1, January 2008 (2008-01), pages 69-73, XP007905687 ISSN: 1540-8167 * abstract * | 1 | INV. A61K31/343 A61P9/06 |
| A | TAFRESHI MOHAMMAD J ET AL: "A review of the investigational antiarrhythmic agent dronedarone" JOURNAL OF CARDIOVASCULAR PHARMACOLOGY AND THERAPEUTICS, vol. 12, no. 1, March 2007 (2007-03), pages 15-26, XP008096445 ISSN: 1074-2484 * the whole document * | 1-7 | |
| A | TOUBOUL PAUL ET AL: "Dronedarone for prevention of atrial fibrillation: a dose-ranging study." EUROPEAN HEART JOURNAL AUG 2003, vol. 24, no. 16, August 2003 (2003-08), pages 1481-1487, XP007905686 ISSN: 0195-668X * the whole document * | 1-7 | TECHNICAL FIELDS SEARCHED (IPC) A61K |

-/--

The present search report has been drawn up for all claims

| Place of search | Date of completion of the search | Examiner |
|---|---|---|
| The Hague | 9 October 2008 | Scheithe, Rupert |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

Europäisches
Patentamt

European
Patent Office

Office européen
des brevets

## EUROPEAN SEARCH REPORT

Application Number

EP 08 29 0532

### DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| A | SINGH BRAMAH N ET AL: "Dronedarone for maintenance of sinus rhythm in atrial fibrillation or flutter." THE NEW ENGLAND JOURNAL OF MEDICINE 6 SEP 2007, vol. 357, no. 10, 6 September 2007 (2007-09-06), pages 987-999, XP007905685 ISSN: 1533-4406 * the whole document * | 1-7 | |
| A | PURERFELLNER H: "ATHENA Studie: Vorläufige Ergebnisse und deren mögliche Auswirkungen auf die antiarrhythmische Pharmakotherapie bei Vorhofflimmern [ATHENA study: Preliminary results and their possible effects on the antiarrhythmic pharmacotherapy in atrial fibrillation]" JOURNAL FUER KARDIOLOGIE, KRAUSE UND PACHERNEGG, PURKERSDORF, AT, vol. 15, no. 7-8, 1 January 2008 (2008-01-01), pages 253-254, XP008096461 ISSN: 1024-0098 * the whole document * | 1-7 | TECHNICAL FIELDS SEARCHED (IPC) |

-/--

The present search report has been drawn up for all claims

| Place of search | Date of completion of the search | Examiner |
|---|---|---|
| The Hague | 9 October 2008 | Scheithe, Rupert |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

Europäisches
Patentamt

European
Patent Office

Office européen
des brevets

## EUROPEAN SEARCH REPORT

Application Number

EP 08 29 0532

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| A | CONNOLLY STUART J ET AL: "Comparison of beta-blockers, amiodarone plus beta-blockers, or sotalol for prevention of shocks from implantable cardioverter defibrillators: the OPTIC Study: a randomized trial" JAMA THE JOURNAL OF THE AMERICAN MEDICAL ASSOCIATION, AMERICAN MEDICAL ASSOCIATION, US, vol. 295, no. 2, 11 January 2006 (2006-01-11), pages 165-171, XP008096671 ISSN: 0098-7484 * abstract * ----- | 1-7 | |

TECHNICAL FIELDS
SEARCHED      (IPC)

The present search report has been drawn up for all claims

| Place of search | Date of completion of the search | Examiner |
|---|---|---|
| The Hague | 9 October 2008 | Scheithe, Rupert |

CATEGORY OF CITED DOCUMENTS

X : particularly relevant if taken alone
Y : particularly relevant if combined with another
    document of the same category
A : technological background
O : non-written disclosure
P : intermediate document

T : theory or principle underlying the invention
E : earlier patent document, but published on, or
    after the filing date
D : document cited in the application
L : document cited for other reasons

 

& : member of the same patent family, corresponding
    document

EPO FORM 1503 03.82 (P04C01)

## REFERENCES CITED IN THE DESCRIPTION

**Patent documents cited in the description**

- EP 0471609 B1

**Non-patent literature cited in the description**

- **Lown B ; Amarasingham R ; Neuman J et al.** New method for treating cardiac arrhythmias; Use of synchronized capacitor discharge. *JAMA,* 1962, vol. 182, 548-555
- **Zipes DP ; Fischer J ; King RM.** Termination of ventricular fibrillation in dogs by depolarizing a critical amount of myocardium. *Am J Cardiol,* 1975, vol. 36, 37-44
- **Botto GL ; Politi A ; Bonini W et al.** External cardioversion of atrial fibrillation: Role of paddle position on technical efficacy and energy requirements. *Heart,* 1999, vol. 82, 726-730
- **Bjerkelund CJ ; Orning OM.** The efficacy of anticoagulant therapy in preventing embolism related to DC electrical conversion of atrial fibrillation. *Am J Cardiol,* 1969, vol. 23, 208-216
- **Arnold AZ ; Mick MJ ; Mazurek RP et al.** Role of prophylactic anticoagulation for direct current cardioversion in patients with atrial fibrillation or atrial flutter. *J Am Coll Cardiol,* 1992, vol. 19, 851-855

- **Rabbino MD et al.** Complications and limitations of direct current countershock. *JAMA,* 1964, vol. 190, 417-420
- **Lown B ; Likoff W ; Dreifus LS.** Cardioversion and digitalis drugs: Changed threshold to electric shock in digitalized animals. *Circ Res,* 1965, vol. 17, 519-531
- **Aberg H ; Cullhed I.** Direct current countershock complications. *Acta Med Scand,* 1968, vol. 183, 415-421
- **Pagan-Carlo, Stone MS ; Kerber RE.** Nature and determinants of skin burns after transthoracic cardioversion. *Am J Cardiol,* 1997, vol. 79, 689-691
- **Lipkin DP ; Frenneaux M ; Stewart R et al.** Delayed improvement in exercise capacity after cardioversion of atrial fibrillation to sinus rhythm. *Br Heart J,* 1988, vol. 59, 572-577
- **Patton JN ; Allen JD ; Pantridge JF.** The effects of shock energy, propranolol, and verapamil on cardiac damage caused by transthoracic countercheck. *Circulation,* 1984, vol. 69, 357-368